# EUROPÄISCHE PATENTSCHRIFT

(11) **EP 2 854 919 B1**
(45) Veröffentlichungstag und Bekanntmachung des Hinweises auf die Patenterteilung: **08.08.2018**
(21) Anmeldenummer: 13725962.8
(22) Anmeldetag: 29.05.2013
(51) Int. Cl.: A61M 16/04, A61M 16/08

(54) **LARYNXMASKENKOPF**
LARYNGEAL MASK
MASQUE LARYNGÉAL

(30) Priorität: 04.06.2012 CH 7682012; 26.07.2012 CH 11692012
(43) Veröffentlichungstag der Anmeldung: 08.04.2015
(73) Patentinhaber: Singularity AG, 8124 Maur (CH)
(72) Erfinder: DUBACH, Werner F., CH-8124 Maur (CH)
(74) Vertreter: Schneider Feldmann AG Patent- und Markenanwälte
(86) Internationale Anmeldenummer: PCT/EP2013/061059
(87) Internationale Veröffentlichungsnummer: WO 2013/182460

(56) Entgegenhaltungen:
- EP-A2- 1 875 937
- US-A- 3 769 983
- US-A- 6 070 581
- US-A- 6 095 144
- US-A1- 2006 102 186
- US-A1- 2011 277 772
- US-B2- 7 546 838
- US-B2- 7 878 201

## Beschreibung

Die vorliegende Erfindung betrifft einen Larynxmaskenkopf mit einer dorsal angeordneten Deckplatte und einem damit verbundenen supraglottischen Tubus, wobei der Larynxmaskenkopf einen ventralen Respirationsraum umgebenden Cuff aufweist.

Es sind eine Vielzahl von Larynxmasken mit verschiedenen Konstruktionsprinzipien bekannt und auf dem Markt erhältlich. Die Mehrzahl aller Larynxmaskenköpfe weisen eine dorsal angeordnete Deckplatte auf, die mit einem supraglottischen Tubus verbunden ist. Auf der ventralen Seite des Larynxmaskenkopfes ist ein Respirationsraum vorhanden, der von einem Cuff umgeben wird. In der Mehrzahl aller bekannten Larynxmaskenköpfe ist dieser Cuff aufblasbar. Typische Beispiele solcher Larnxmasken deren Larynxmaskenkopf eine dorsal angeordnete Deckplatte und einen ventralen Respirationsraum haben, wobei der Respirationsraum mit einem aufblasbaren Cuff umgeben ist, zeigen beispielsweise die US 5,878,745, die US 2003/0037790 oder auch die US 7,040,322. Larynxmasken deren Larynxmaskenkopf einen nicht aufblasbaren Cuff besitzen sind wesentlich seltener. Solche Larynxmasken sind meistens einstückig ausgeführt, indem der Larynxmaskenkopf und der supraglottische Tubus einstückig gebildet sind und in etwa die Form eines Handduschkopfes haben. Bereits aus der EP 0 389272 ist eine Larynxmaske bekannt die auch mit einem nichtaufblasbaren Cuff gestaltet sein kann, wobei dieser Cuff eine zur ventralen Richtung gelegene, umlaufende Dichtlippe aufweist, um die Abdichtung zu verbessern. Auch die GB 2404863 zeigt eine Larynxmaske, wobei hier die Larynxmaske und der supraglottische Tubus einstückig miteinander verbunden sind. Die formliche Anpassung dieser Larynxmaske erfolgt auch hier, wie in der zuvor genannten EP 389272, durch eine umlaufende Dichtlippe, die am Cuff angeformt ist.

Demgegenüber zeigt die EP 1875937 eine gleichartige Larynxmaske wie in der zuvor genannten GB 2404863, wobei hier die Dichtung im Wesentlichen durch eine verbesserte anatomisch angepassten Form realisiert wird. Hierbei wird vorgeschlagen, den nicht aufblasbaren Cuff aus einem Material mit einer Shore Härte von 0 - 20, insbesondere mit einer Härte von 4 - 12 Shore A Härte zu gestalten. Die Dichtung des Cuffs erfolgt somit nur mittels der Weichheit des Cuffs. Die erforderliche Stabilität während der Einführung gewährleistet eine härtere Deckplatte mit Löchern in der Spitze der Deckplatte, um die Biegsamkeit der Deckplatt in diesem Bereich zu gewährleisten.

Ferner zeigt das Dokument US 6 070 581 A einen Larynxmaskenkopf bestehend aus einer Deckplatte mit supraglottischem Tubus, der durch die Deckplatte hindurch und durch ein Dichtelement aus offenem oder geschlossenem Schaumgummi in Form eines Polsters oder Kissens führt.
Wie bei der EP 1 875 937 A2 erfolgt die Anpassung nur durch die Komprimierbarkeit des Materials.

Es ist daher die Aufgabe der vorliegenden Erfindung einen Larynxmaskenkopf mit Deckplatte gemäss Oberbegriff des Anspruchs 1 zu schaffen, der spritzgusstechnisch einstückig, aus einem einzigen Material fertigbar ist und eine formliche Anpassung in lateral-medialer und dorsal-ventraler Richtung nur durch formgebende Mittel zulässt. Diese Aufgabe löst ein Larynxmaskenkopf mit den Merkmalen des kennzeichnenden Teils des Patentanspruches 1.

Diese Grössenanpassung erfolgt dadurch, dass die nicht-pneumatischen Mittel offene Rinnen sind, wobei die Rinnen zur medial-lateralen Grössenanpassung eine ventral-dorsale und die zur ventral-dorsalen Grössenanpassung dienenden Rinnen eine medial-laterale Eindringrichtung bzw. Tiefenerstreckung haben und diese Rinnen beidseitig der Mittelachse des Larynxmaskenkopfes angeordnet und in Verlaufsrichtung des Cuffs sich erstrecken.

Die federnde Wirkung der Rinnen ergibt sich einerseits durch die Materialauswahl und andererseits durch die Bestimmung der Wandstärke der Seitenwände der Rinne. Sowohl in Bezug auf die Materialwahl wie auch in Bezug auf die Wandstärkenausgestaltung ist man jedoch an gewissen Gegebenheiten gebunden und entsprechend werden bevorzugt federnd abstützende Organe vorgeschlagen, mittels der diese Federkraft der Rinnen beeinflusst werden kann. Diese Organe sind in den weiteren Ansprüchen dargelegt.

Selbstverständlich kann ein solcher Larynxmaskenkopf auch einstückig mit dem damit verbundenen supraglottischen Tubus gestaltet sein.

In der Zeichnung ist ein bevorzugtes Ausführungsbeispiel des Erfindungsgegenstandes dargestellt und anhand der nachfolgenden Beschreibung, mit Bezug auf die beiliegenden Zeichnungen, beschrieben. Es zeigen:
- Fig. 1: zeigt eine Larnxmasken in der Gesamtansicht von oben auf die Deckplatte, und
- Fig. 2: dieselbe Larnxmaske in einer Seitenansicht, während
- Fig. 3: einen Vertikalschnitt durch den Larynxmaskenkopf entlang der Linie A-A wie Figur 1 zeigt.
- Fig. 4-7: stellen systematische Schnittzeichnungen durch die Rinne dar mit unterschiedlichen Mittel zur medial-lateralen Grössenanpassung, während die
- Fig. 8-11: Teilansichten von Rinnen darstellen, mit unterschiedlich gestalteten Stützwänden.
- Fig. 12: zeigt einen vergrösserten Vertikalschnitt durch eine erfindungsgemässe Variante des Larynxmaskenkopfes mit Mittel zur medial-lateraler Grössenanpassung, während
- Fig. 13: eine weitere Variante des Larynxmaskenkopfes mit Mittel zur ventral-dorsalen Grössenanpassung in der Seitenansicht, in
- Fig. 14: in der Aufsicht und
- Fig. 15: in einem Vertikalschnitt entlang der Linie C-C in Figur 14. Eine weitere Variante zeigt die
- Fig. 16: die an sich baugleich der Ausführung nach Figur 13-15 ist, mit jedoch zwei parallelen Nuten in der Seitenansicht und
- Fig. 17: wiederum in der Aufsicht und
- Fig. 18: abermals als Vertikalschnitt entlang der Linie C-C in Figur 17. Eine letzte Variante der Erfindung zeigt die
- Fig. 19: den Larynxmaskenkopf mit einstückig angeformtem Tubus in perspektivischer Darstellung und
- Fig. 20: in einer Seitenansicht und
- Fig. 21: mit Blickrichtung auf die Deckplatte.

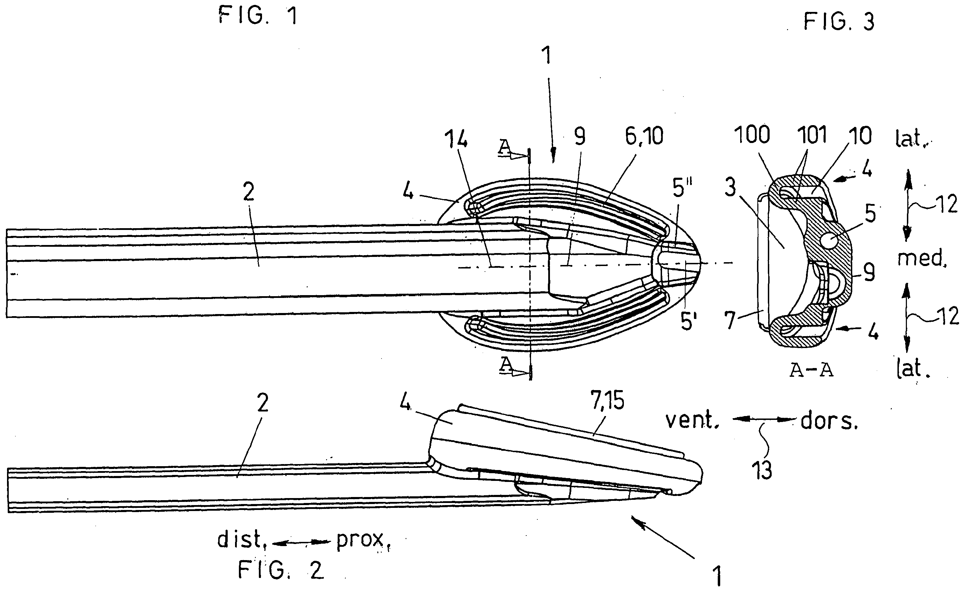

Die Figuren 1 - 3 zeigen eine erfindungsgemäss ausgestaltete Larynxmaske. Diese besteht aus den beiden Hauptbestandteilen, nämlich dem Larynxmaskenkopf 1 und dem daran angeschlossenen supraglottischen Tubus 2. Diese beiden Teile können entweder einzeln gefertigt oder zusammen einstückig gefertigt sein. Im Blick auf die dorsale Seite des Larynxmaskenkopfes 1, wie in der Figur 1 dargestellt, erkennt man mittig deren Deckplatte 9, die hier einstückig Bestandteil des Larynxmaskenkopfes ist und an dem anschliessend der supraglottische Tubus 2 folgt. Unter der Deckplatte 9, und damit in ventraler Richtung, befindet sich ein Respirationsraum 3. Dieser Respirationsraum 3 wird allseitig begrenzt durch einen umlaufenden Cuff 4. In der Deckplatte 9 verläuft ein Ösophagealdurchgang 5. Dies ist selbstverständlich nur eine optionale Möglichkeit und für die Realisation der Erfindung nicht wesentlich. Dieser Ösophagealdurchgang 5 tritt bei 5' in den Larynxmaskenkopf 1 ein. Entsprechend ist ein offener Kanal vorhanden, der über den Cuff 4 hinwegführt. Seitlich dieses Kanals sind Stützen 5' vorhanden, die den Cuff in diesem Bereich versteifen und somit ein Knicken des Larynxmaskenkopfes 1 während der Einführung vorbeugt. Auf beiden Seiten der Deckplatte 9 verlaufen im Cuff 4 eingelassene Mittel 6 die zur medial-lateralen Grössenanpassung dienen. Solche Mittel zur Grössenanpassung in medial-lateraler Richtung können beispielsweise auch, hier nicht dargestellt, umlaufende und abstehende Rippen sein, doch bevorzugterweise, und hier auch dargestellt, handelt es sich bei diesen Mittel 6 zur medial-lateralen und / oder dorsal-ventralen Grössenanpassung um entsprechend im Cuff 4 und deren Verlaufrichtung sich erstreckende Rinnen 10. Diese Rinnen 10 verlaufen im hier gezeigten Ausführungsbeispiel vom Bereich der Einführung des Tubuses 2 in den Larynxmaskenkopf 1 bis in den Bereich in dem der Ösophagealdurchgang 5 austritt und über den Cuff hinweg ausgeführt wird. Die Eindringrichtung der Rinnen 10 verläuft in diesem Beispiel in ventral-dorsaler Richtung. Ist der Larynxmaskenkopf 1 ohne Ösophagealdurchgang gestaltet, so könnte im Prinzip sich die Rinne 10 auch um die Deckplatte 9 herum erstrecken. Bevorzugterweise wird dies jedoch vermieden, um dadurch die Spitze des Larynxmaskenkopfes 1 nicht zu schwächen. Zudem ist auch eine Grössenanpassung in distal-proximaler Richtung nicht erforderlich.

Die beidseitig der Deckplatte 9 verlaufenden Rinnenabschnitte haben in etwa eine teilelliptische Bahnführung. Wenn nachfolgend, im Bezug auf diese Ausführungsform gemäss den Figuren 1 - 3, Figur 12 und Figuren 19 - 21 von der Rinne 10 gesprochen wird, so werden hierunter auch die beiden Rinnenabschnitte beidseits der Deckplatte 9 verstanden. Hier wird auch von beidseitig der Mittelachse 14 gesprochen, da in der Aufsicht der Larynxmaskenkopf 1 in etwa hierzu eine symmetrische Form aufweist.

Diese Rinnen 10 stellen, wie bereits erwähnt, hier die Mittel 6 zur medial-lateralen Grössenanpassung dar. Diese Richtung ist mit dem Doppelpfeil 12 symbolisch dargestellt. Ein in Verlaufrichtung zu diesem Pfeil senkrecht verlaufender Doppelpfeil 13 zeigt den ventral-dorsalen Richtungsverlauf, und in dieser Richtung führt das Mittel 7 zur Anpassung in ventral-dorsaler Richtung. Dieses Mittel 7 steht im hier gezeigten Beispiel aus einer umlaufenden, federnden Dichtlippe 15. Solche federnden Dichtlippen zur dichtenden, federnden Anpassung in ventral-dorsaler Richtung sind auch aus den eingangs erwähnten Dokumenten bereits bekannt.

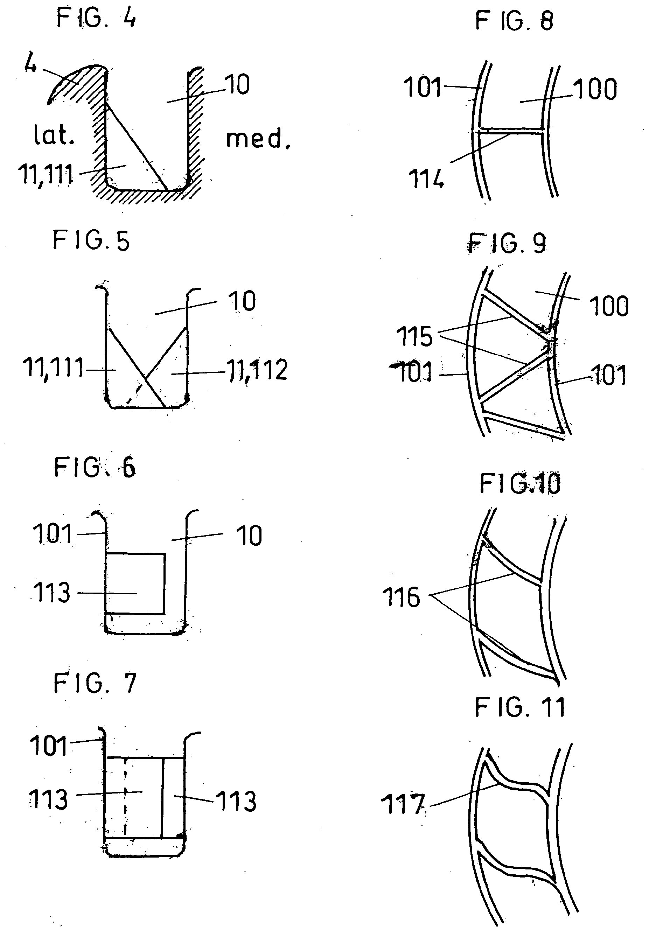

Die Federwirkung der Mittel zur 6 lässt sich durch die Wahl des Materiales, d.h. der Wahl des hier eingesetzten Kunststoffes, bestimmen, aber auch durch die geometrische Auslegung, nämlich durch die Wandstärke der Rinne 10. Diese Rinne 10 besitzt einen Boden 100 und Seitenwände 101. Sowohl die Dicke des Bodens als auch der Seitenwände lässt sich selbstverständlich fast beliebig festlegen, und entsprechend bildet sich die Weichheit oder Härte der Federwirkung des Mittels 6 zur medialen-lateralen Grössenanpassung. Trotzdem aber ist man gerade im medizinaltechnischen Bereich nicht vollständig frei Materialien so zu wählen wie man das gerne wünschen würde, und auch in Bezug auf die Geometrie ist man natürlich an anatomischen Verhältnissen gebunden. Um trotzdem die Federwirkung in lateral-medialer oder dorsal-ventraler Richtung einstellen zu können, werden verschiedene, federnde, abstützende Organe 11 vorgeschlagen und anhand der Figuren 4 - 11 erläutert.

In der Beschreibung der nachfolgenden vereinfachten Darstellung der Rinne wird von lateralen oder medialen Seitenwänden gesprochen, entsprechend der hier gezeigten Lage. Analog würde man bei einer liegend dargestellten Rinne von dorsalen und ventralen Seitenwänden sprechen.

Eine erste Variante ist in der Figur 4 gezeigt. Die Rinne 10 ist im Querschnitt rein symbolisch dargestellt. Diese U-förmige Rinne 10 besitzt nun, wie erwähnt, ein federndes, abstützendes Organ 11. Dieses Organ 11 besteht nun in der Ausführungsform gemäss der Figur 4 aus einer Stützrippe 111, die sich vom Boden 100 der Rinne 10 zu einer der Seitenwände 101 hin geneigt nach oben erstreckt. Je nachdem welche Federcharakteristik man hiermit erzeugen will stützt sich die Stützrippe 111 an jener Seitenwand 101 die näher zur Mitte, also in radialer Richtung angeordnet ist, oder an der gegenüberliegenden Seitenwand, welche die laterale Seitenwand darstellt. Wird nur die laterale Seitenwand gestützt, so wird diese entsprechend steifer und bildet so einen erhöhten Federdruck in lateraler Richtung, oder man ordnet die Stützrippen 111 auf der medialen Seite an, wodurch jene Seitenwand versteift wird, während die laterale Seitenwand entsprechend leicht verformbar sein kann.

Es ist aber durchaus möglich, auch an beiden Seitenwänden solche Stützrippen 111, 112 anzuordnen, wie dies symbolisch die Figur 5 zeigt. Hierdurch wird insbesondere die Rinne 10 im Bereich von dessen Boden 100 eine erhöhte Steifigkeit erzielen, während der obere Bereich bzw. der dorsale Bereich der Rinne praktisch unversteift ist.

Die Variante nach der Figur 6 zeigt eine Möglichkeit, bei der als federnd abstützendes Organ 111 ein Stützlappen 113 angeformt ist, der sich von einer Seitenwand in Richtung zur anderen Seitenwand hin erstreckt, aber die Rinne nicht vollständig quert. Damit eine solch stützende, querende Stützrippe 113 Sinn macht, sollte sie mindestens 50% der Rinnenbreite überqueren. Ein solcher Stützlappen 113 ist lediglich an der Seitenwand angeformt, nicht jedoch am Boden. Damit bleibt der Boden flexibel. Nachteilig bei dieser Lösung ist jedoch die relativ komplexe Fertigung, da dies eine Spritzgussform mit entsprechenden Schiebern oder einem kollabierbaren Kern voraussetzt.

Eine Lösung derselben Art, jedoch mit Stützlappen 113 die abwechslungsweise an der einen und an der anderen Seitenwand 101 angeformt sind, zeigt die Figur 7.

Während in den Figuren 4 - 7 die Rinne 10 immer im Querschnitt symbolisch gezeigt ist, sind in den Ausführungsvarianten gemäss den Figuren 8 - 11 diese Rinne in der Aufsicht von oben gezeigt, und selbstverständlich nur ein Teilbereich der Rinne. Hier sieht man somit flach auf den Boden 100 und die Seitenwände 101 sind wiederum rein symbolisch mit einer Doppellinie angegeben. In der Figur 8 ist nun als federndes, abstützendes Organ 11 eine Lösung mit einer Stützwand 114 dargestellt. Diese Stützwand 114 ist nun sowohl am Boden 100 als auch an den beiden Seitenwänden angeformt. In der Variante nach der Figur 8 verläuft hierbei die Stützwand 114 senkrecht zur Verlaufsrichtung der Rinne 10 im entsprechenden Bereich. Alternativ ist in der Figur 9 eine Lösung dargestellt, wobei die Stützwände 15 nun geneigt zu dieser Verlaufsrichtung angeordnet sind. Aber selbstverständlich sind auch diese Stützwände 115 sowohl an den beiden Seitenwänden 101 wie auch am Boden 100 angeformt.

Solche Stützwände brauchen selbstverständlich keineswegs immer geradlinig zu verlaufen. So zeigt die Figur 10 eine Variante bei der gekrümmt verlaufende Stützwände 116 vorhanden sind. Alternativ können diese Stützwände auch S-förmig verlaufen, wie dies die Figur 11 zeigt, während die geradlinig verlaufenden Stützwände praktisch eine höhere Steifigkeit der Seitenwände 101 der Rinne 10 bewirken, da erst nach einer gewissen eingetretenen Verformung sich diese Stützwände in ihrer Form verändern und danach sich weiter verformen lassen. Dieses Problem ergibt sich bei den gekrümmt verlaufenden Stützwänden 116 und 117 nicht.

Da der Larynxmaskenkopf 1 insgesamt aus Kunststoff gefertigt ist ergeben sich bei der Einführung der Larynxmaske auch Relativverschiebungen zwischen den Seitenwänden 101 der Rinne 10. Dies kann zu gewissen Blockierungen in ungünstigen Fällen führen, die eine Federbewegung nur unter erschwerten Bedingungen erlaubt. Bei der Wahl der gekrümmten Stützwände in S-Form, wie dies die Stützwände 117 besitzen, ergibt sich diese Problematik nicht.

Obwohl hier nicht dargestellt, können selbstverständlich statt lediglich einer Rinne 10 jeweils auf einer Seite der Deckplatte im Cuff 4 auch zwei oder mehrerer solcher Rinnen parallel verlaufend angeordnet sein. Bei einer solchen Lösung würde man dann die Rinnen unterschiedlich lang gestalten. Hierbei würde man die Rinnen in medialer Richtung näher der Mittelachse 14 kürzer und die weiter aussen in lateraler Richtung angeordneten, parallelen Rinnen 10 länger gestalten. Dies ergibt eine höher Steifigkeit im Bereich nahe dem Respirationsraum, während weiter aussen in lateraler Richtung die Flexibilität erhöht ist.

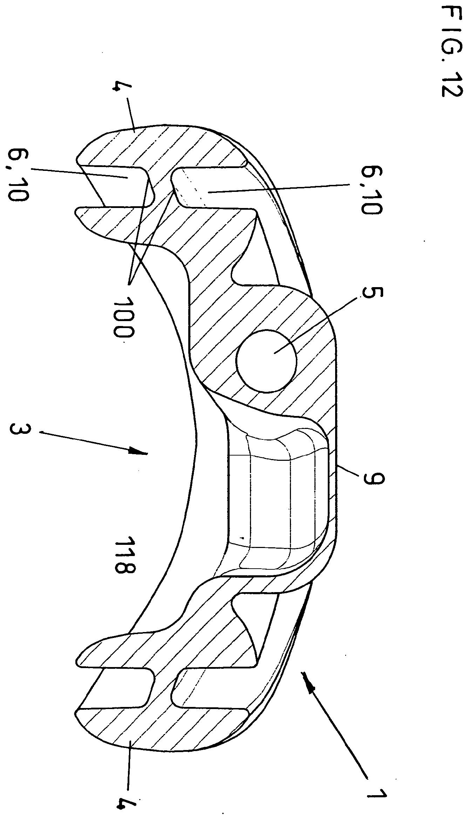

Die Ausführungsform gemäss der Figur 12 entspricht im Wesentlichen der Ausführung gemäss den Figuren 1 -3. In diesem vergrösserten Vertikalschnitt durch den Larynxmaskenkopf 1 erkennt man zu oberst die Deckplatte 9 und den darunter verlaufenden Ösophagealdurchgang 5. Um die Deckplatte 9 herum verläuft der Cuff 4. Im Gegensatz zur zuvor beschriebenen Ausführungsform ist als Mittel zur medial-lateralen Grössenanpassung nicht nur eine einzige Rinne 10 vorhanden, sondern zwei Rinnen, die fluchtend übereinander angeordnet sind und einen gemeinsamen Boden 100 haben. Hier ist allerdings nicht nur eine, von der dorsalen Richtung her, eindringende Rinne 10 vorhanden, sondern auch eine von der ventralen Seite her eindringende Rinne 10 vorhanden. Da diese beiden Rinnen 10 fluchtend übereinander gestellt sind, haben sie einen gemeinsamen Verbindungswandbereich als Boden 100. Die Verbindungswand 118 ist bevorzugterweise nicht exakt medial-lateral verlaufend, sondern leicht geneigt, um damit die Verformung bei medial-lateral wirkendem Druck zu erleichtern.

Der Cuff 4 ist somit auf der ventralen, dichtend aufzuliegenden Seite zweigeteilt. Diese Zweiteilung ist besonders vorteilhaft, da hierdurch praktisch zwei Dichtflächen entstehen und dank der kleineren Auflageflächen ein höherer spezifischer Druck an den Dichtkanten erzielt wird, womit eine erhöhte Abdichtwirkung realisiert wird.

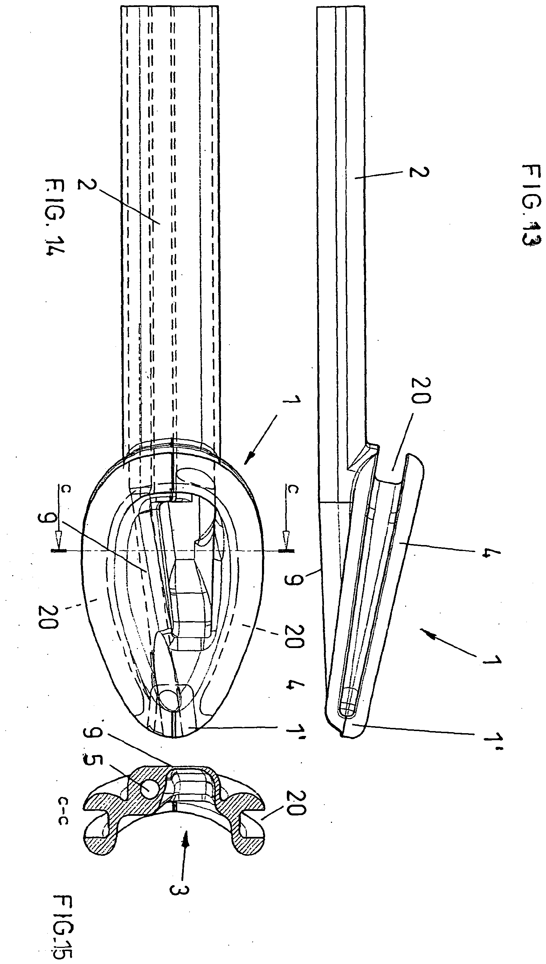

Während die bisherigen Ausführungsformen allesamt Lösungen gezeigt haben mit Mittel 6 zur medial-lateralen Grössenanpassung, sind in den nachfolgenden Figuren Lösungen gezeigt mit Mittel 7 zur ventral-dorsalen Grössenanpassung. Diese Mittel 7 werden hier wiederum als Rinnen realisiert, doch werden diese Rinnen, die eine lateral-mediale Eindringrichtung in den Cuff aufweisen mit 20 bezeichnet. Diese Rinnen 20 mit lateral-medialer Eindringrichtung bewirken eine ventral-dorsale Grössenanpassung. Auch hier erkennt man den Larynxmaskenkopf 1 mit dem daran angeformten supraglottischen Tubus 2, der wie Figur 15 erkennen lässt unter der Deckplatte 9 den ventralen Respirationsraum 3 und den Ösophagealdurchgang 5 aufweist. Die Rinne 20 verläuft hufeisenförmig im Cuff 4. Lediglich im Bereich der Spitze 1' ist die Rinne 20 nicht durchgezogen. Dies ist an sich zwar möglich, doch bevorzugterweise wird darauf verzichtet, um so eine höhere Festigkeit der Spitze 1' zu erhalten und damit wird sichergestellt, dass diese Spitze 1' keiner Knickwirkung Vorschub leistet.

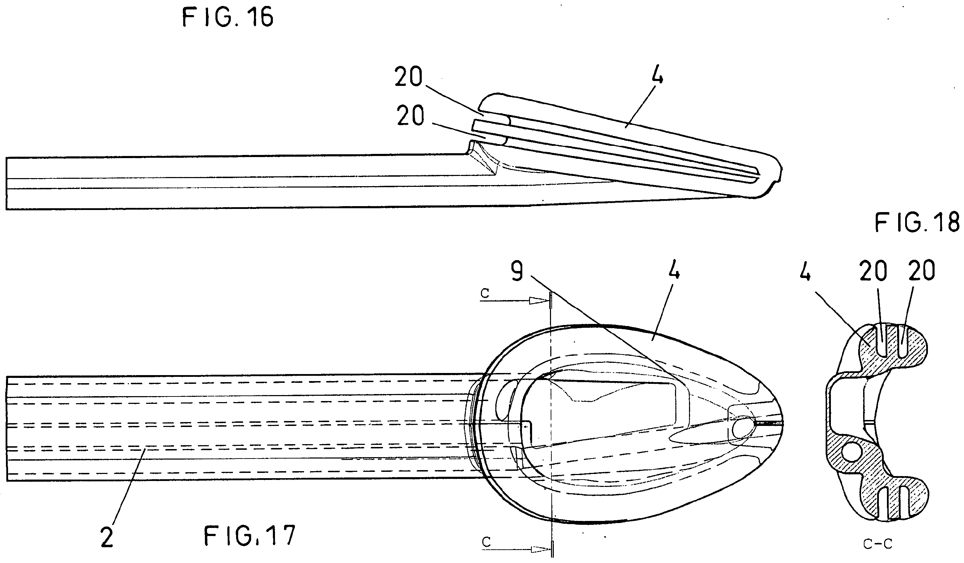

In der Ausführungsform gemäss den Figuren 16 - 17 sind zwei parallel zueinander verlaufende Rinnen 20 verlaufen. Ansonsten ist diese Lösung identisch mit der Variante gemäss den Figuren 13 - 15. Auf die Beschreibung der gleichbleibenden Elemente wird daher verzichtet. Wie bereits zuvor erwähnt, führen die mehreren Dichtkanten zu einem erhöhten spezifischen Druck und damit zu einer erhöhten Dichtigkeit.

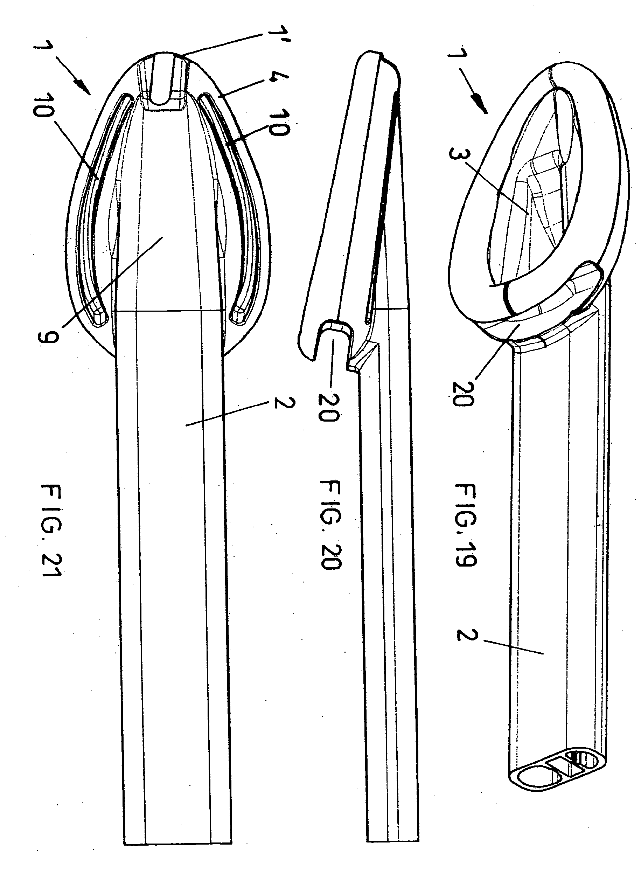

Letztlich ist in der Ausführungsform gemäss den Figuren 19 bis 21 eine kombinierte Lösung dargestellt. In der perspektivischen Ansicht gemäss der Figur 19 ist nur eine Teilrinne mit in lateral-medialer Richtung verlaufenden Eindringtiefe ersichtlich. Diese Rinne 20 ist lediglich im Bereich der Einführung des supraglottischen Tubuses 2 in den Larynxmaskenkopf 1 angebracht. Angesichts der hier erhöhten Materialkonzentration wäre ansonsten dieser Bereich relativ steif. Die Rinnen 10 entsprechen in Grösse, Verlauf und Anordnung jener Variante, die bereits anhand der Figuren 1 - 3 beschrieben worden sind. Auch bei diesen kombinierten Lösungen ist es durchaus denkbar, dass auch hier mehrere parallel zueinander verlaufende Rinnen 10 beziehungsweise parallel zueinander verlaufenden Rinnenabschnitte 20 vorhanden sein können.

Dank der Vielzahl der möglichen Varianten, die selbstverständlich auch noch mit entsprechenden Stützrippen, Stützlappen oder gerade oder gekrümmt verlaufenden Stützwände versehen sein können, ergibt sich eine Vielzahl von Kombinationsmöglichkeiten, die praktisch jede beliebige Federcharakteristik des Cuffs erzielbar machen. Die Wahl der entsprechenden Kombination beziehungsweise Ausgestaltung des Larynxmaskenkopfes mit einer oder mehreren Rinnen 10, mit ventral-dorsaler Eindringrichtung oder einer oder mehrerer parallel verlaufenden Rinnen 20 mit lateral-medialer Eindringrichtung gibt dem Hersteller die Möglichkeit mit relativ geringen Formänderungen eine Vielzahl von Varianten auf dem Markt anzubieten.

### Bezugszeichenliste:

- 1: Larynxmaskenkopf
- 1': Spitze des Larynxmaskenkopfes
- 2: supraglottischer Tubus
- 3: ventraler Respirationsraum
- 4: umgebender Cuff
- 5: Ösophagealdurchgang
- 5": seitliche Stützen
- 5': Ösophagealeingang
- 6: Mittel zur medial-lateralen Grössenanpassung
- 7: Mittel zur ventral-dorsalen Grössenanpassung
- 8: umlaufende Dichtlippe
- 9: Deckplatte
- 10: Rinne mit ventral-dorsaler Eindringrichtung
- 11: federnd abstützende Organe
- 12: lateral-mediale Richtung
- 13: ventral-dorsale Richtung
- 14: Mittelachse
- 15: ventral-dorsal federnde umlaufende Dichtlippe
- 20: Rinne mit lateral-medialer Eindringrichtung
- 100: Boden der Rinne
- 101: Seitenwand der Rinne
- 111: Stützrippe einseitig
- 112: Stützrippe beidseitig
- 113: Stützlappen
- 114: Stützwand gerade
- 115: Stützwand geneigt
- 116: Stützwand gekrümmt
- 117: Stützwand S-förmig
- 118: Verbindungswand

## Patentansprüche

1. Larynxmaskenkopf (1) aus Kunststoff mit einer dorsal angeordneten Deckplatte (9) und einem damit verbundenen supraglottischen Tubus (2), wobei der Larynxmaskenkopf (1) einen ventralen Respirationsraum (3) umgebenden Cuff (4) aufweist, der lediglich nichtpneumatische Mittel (6, 7) aufweist, zur flexiblen Grössenanpassung in medial-lateraler und / oder in dorsal-ventraler Richtung (12), **dadurch gekennzeichnet, dass** die nicht-pneumatischen Mittel (6) offene Rinnen (10, 20) sind, wobei die Rinnen (10) zur medial-lateralen Grössenanpassung eine ventral-dorsale und die zur ventral-dorsalen Grössenanpassung dienenden Rinnen (20) eine medial-laterale Eindringrichtung bzw. Tiefenerstreckung haben, wobei die Rinnen (10, 20) zur Grössenanpassung im Cuff (4) beidseitig der Mittelachse (14) des Larynxmaskenkopfes (1) angeordnet und in Verlaufsrichtung des Cuffs (4) sich erstrecken.

2. Larynxmaskenkopf (1) nach Anspruch 1, **dadurch gekennzeichnet, dass** in jeder Rinne (10, 20) je mindestens eine, die Rinne (10, 20) mindestens teilweise querende und deren Seitenwände federnd abstützende Organe (11) eingeformt sind.

3. Larynxmaskenkopf (1) nach Anspruch 2, **dadurch gekennzeichnet, dass** die federnd abstützenden Organe (11) Stützrippen (111) sind, welche vom Boden (100) der Rinne (10) sich zu einer Seitenwand (101) hin geneigt zur Öffnung hin nach oben erstrecken.

4. Larynxmaskenkopf (1) nach Anspruch 3, **dadurch gekennzeichnet, dass** sich abwechslungsweise jeweils eine Stützrippe (111) zur lateralen oder ventralen und darauf eine (112) zur medialen oder dorsalen Seitenwand (101) der Rinne (10) hin geneigte zur Öffnung hin nach oben erstreckt.

5. Larynxmaskenkopf (1) nach Anspruch 2, **dadurch gekennzeichnet, dass** die Organe (11) ein, die Rinne (10, 20) von einer Seitenwand (101) her in Richtung zur gegenüberliegenden Seitenwand (10) hin zumindestens 50% bis 99% die Rinne querende Stützlappen (113) sind.

6. Larynxmaskenkopf (1) nach Anspruch 5, **dadurch gekennzeichnet, dass** die Stützlappen (113) abwechselnd an der einen und an der anderen Seitenwand (101) angeformt sind.

7. Larynxmaskenkopf (1) nach Anspruch 2, **dadurch gekennzeichnet, dass** die federnd die Seitenwände (101) abstützenden Organe (11) Stützwände (114 - 117) sind, die an beiden Seitenwände (101) und am Boden (100) der Rinne (10) angeformt sind.

8. Larynxmaskenkopf (1) nach Anspruch 7, **dadurch gekennzeichnet, dass** die Stützwände (114, 115) gerade oder gekrümmt, vorzugsweise s-förmig verlaufen und die Rinne (10) senkrecht (114) und/oder geneigt (115) zur Längserstreckungsrichtung derselben queren.

9. Larynxmaskenkopf (1) nach Anspruch 7, **dadurch gekennzeichnet, dass** die Stützwände (115) unterschiedlich geneigt zur Längserstreckungsrichtung der Rinne (10) diese queren.

10. Larynxmaskenkopf (1) nach Anspruch 2, **dadurch gekennzeichnet, dass** die die Seitenwände (101) der Rinne (10, 20) und die federnd stützenden Organe (11) in der Wandstärke der zu erzielenden Federkraft entsprechend wählbar sind.

11. Larynxmaskenkopf (1) nach Anspruch 1, **dadurch gekennzeichnet, dass** beidseitig der Mittelachse (14) des Larynxmaskenkopfes (1) je zwei oder mehr parallel zueinander verlaufende Rinnen (10, 20) eingeformt sind.

12. Larynxmaskenkopf (1) nach Anspruch 1, **dadurch gekennzeichnet, dass** im Cuff (4) mindestens zwei fluchtend übereinander in ventral-dorsaler Richtung in den Cuff eindringende Rinnen (10) angebracht sind.

13. Larynxmaskenkopf (1) nach Anspruch 1, **dadurch gekennzeichnet, dass** die Rinnen (20) mit im wesentlichen medial-lateraler Eindringrichtung hufeisenförmig in der Erstreckungsrichtung des Cuffs )4) verlaufen, und wobei die Spitze (1') des Cuffs (4) rinnenfrei ist.

14. Larynxmaskenkopf (1) nach Anspruch 1, **dadurch gekennzeichnet, dass** im Bereich in dem der Tubus (2) den Cuff (4) quert mindestens eine Rinne (10) zur ventral-dorsalen Grössenanpassung vorhanden ist, während anschliessend in Richtung zur Spitze (1') beidseitig der Deckplatte mindestens je eine Rinne (10) zur ventral-dorsalen Grössenanpassung im Cuff verläuft.

## Claims

1. A laryngeal mask head (1) consisting of plastic with a dorsal cover plate (9) and a supraglottic tube (2) connected thereto, the laryngeal mask head (1) having a cuff (4) which surrounds a ventral respiration chamber (3) and has only non-pneumatic means (6, 7) for flexible size adjustment in the medial-lateral and/or dorsal-ventral direction (12),
**characterised in that**
the non-pneumatic means (6) are open grooves (10, 20), wherein the grooves (10) for medial-lateral size adjustment have a ventral-dorsal penetration direction or depth and the grooves (20) for ventral-dorsal size adjustment have a medial-lateral penetration direction or depth, wherein the grooves (10, 20) for size adjustment are arranged in the cuff (4) on both sides of the centre axis (14) of the laryngeal mask head (1) and extend in the contour direction of the cuff (4).

2. The laryngeal mask head (1) according to Claim 1,
**characterised in that**
in each groove (10, 20) is formed at least one element (11) which at least partially crosses the groove (10, 20) and resiliently supports the side walls thereof.

3. The laryngeal mask head (1) according to Claim 2,
**characterised in that**
the resiliently supporting elements (11) are supporting ribs (111) which extend upwards from the bottom (100) of the groove (10) towards a side wall (101) in an inclined manner towards the opening.

4. The laryngeal mask head (1) according to Claim 3,
**characterised in that**
in an alternating manner, one supporting rib (111) extends upwards towards the lateral or ventral side wall (101) of the groove (10) and then one supporting rib (112) extends upwards towards the medial or dorsal side wall of the groove, in an inclined manner towards the opening.

5. The laryngeal mask head (1) according to Claim 2,
**characterised in that**
the elements (11) are a supporting lobe (113) which crosses at least 50% to 99% of the groove (10, 20) from one side wall (101) towards the opposite side wall (10).

6. The laryngeal mask head (1) according to Claim 5,
**characterised in that**
the supporting lobes (113) are formed alternately on one side wall and on the other side wall (101).

7. The laryngeal mask head (1) according to Claim 2,
**characterised in that**
the elements (11) which resiliently support the side walls (101) are supporting walls (114-117) which are formed on both side walls (10) and on the bottom (100) of the groove (10).

8. The laryngeal mask head (1) according to Claim 7,
**characterised in that**
the supporting walls (114, 115) are straight or curved, preferably S-shaped, and run the groove (10) perpendicularly (114) and/or in an inclined manner (115) to the longitudinal direction of same.

9. The laryngeal mask head (1) according to Claim 7,
**characterised in that**
the supporting walls (115) cross the groove (10) at different inclinations to the longitudinal direction of the groove.

10. The laryngeal mask head (1) according to Claim 2,
**characterised in that**
the wall thickness of the side walls (101) of the groove (10, 20) and of the resiliently supporting elements (11) can be selected according to the spring force to be achieved.

11. The laryngeal mask head (1) according to Claim 1,
**characterised in that**
in each case two or more parallel grooves (10, 20) are formed on both sides of the centre axis (14) of the laryngeal mask head (1).

12. The laryngeal mask head (1) according to Claim 1,
**characterised in that**
at least two grooves (10) which penetrate the cuff in alignment one above the other in the ventral-dorsal direction are made in the cuff (4).

13. The laryngeal mask head (1) according to Claim 1,
**characterised in that**
the grooves (20) with a substantially medial-lateral penetration direction run in a horseshoe shape in the extension direction of the cuff (4), and the tip (1') of the cuff (4) has no grooves.

14. The laryngeal mask head (1) according to Claim 1,
**characterised in that**
in the region in which the tube (2) crosses the cuff (4) there is at least one groove (10) for ventral-dorsal size adjustment, while adjacent thereto in the direction towards the tip (1') at least one groove (10) for ventral-dorsal size adjustment runs in the cuff on both sides of the cover plate.

## Revendications

1. Tête de masque laryngé (1) en matière plastique comportant une plaque de recouvrement disposée au dos (9) et un tube supraglottique (2), la tête de masque laryngé (1) présentant un manchon (4) entourant une chambre de respiration ventrale (3) qui présente simplement des moyens non pneumatiques (6, 7) permettant l'adaptation flexible de la taille dans le sens médial-latéral et/ou dans le sens dorsal-ventral (12),
**caractérisée en ce que** les moyens non pneumatiques (6, 7) sont des rainures ouvertes (10,20), les rainures (20) permettant l'adaptation médiale-latérale de la taille présentant un sens de pénétration ou une extension en profondeur ventrale-dorsale et celles (20) permettant une adaptation ventrale dorsale un sens de pénétration ou une extension en profondeur médiale-latérale, les rainures (10,20) permettant l'adaptation de la taille étant disposées dans le manchon (4) des deux côtés de l'axe central (14) de la tête de masque laryngé (1) et s'étendant dans le sens d'orientation du manchon (4).

2. Tête de masque laryngé (1) selon la revendication 1,
**caractérisée en ce que**, dans chaque rainure (10,20), est formé chaque fois au moins un organe (11) traversant au moins partiellement la rainure (10,20) et soutenant au moins partiellement élastiquement ses parois latérales.

3. Tête de masque laryngé (1) selon la revendication 2,
**caractérisée en ce que** les organes de soutien élastique (11) sont des nervures de soutien (111) qui s'étendent depuis le fond (100) de la rainure (10) vers une paroi latérale (101) en s'inclinant vers le haut en direction de l'ouverture.

4. Tête de masque laryngé (1) selon la revendication 3,
**caractérisée en ce que** s'étendent alternativement, respectivement une nervure de soutien (111) de la paroi latérale ou ventrale et par-dessus une autre nervure de soutien (112) de la paroi latérale médiale-dorsale (101) de la rainure (10), en s'inclinant vers le haut en direction de l'ouverture.

5. Tête de masque laryngé (1) selon la revendication 2,
**caractérisée en ce que** les organes (11) sont une languette d'appui (113) traversant la rainure (10,20) depuis une paroi latérale (101) en direction de la paroi latérale opposée (10) en traversant au moins 50 à 99 % de la rainure.

6. Tête de masque laryngé (1) selon la revendication 5,
**caractérisée en ce que** les languettes d'appui (113) sont formées alternativement sur l'une et l'autre paroi latérale (101).

7. Tête de masque laryngé (1) selon la revendication 2,
**caractérisée** en ce les organes (11) de soutien élastique des parois latérales (101) sont des parois de soutien (114 à 117) qui sont formées au niveau des deux parois latérales (101) et du fond (100) de la rainure (10).

8. Tête de masque laryngé (1) selon la revendication 7,
**caractérisée en ce que** les parois de soutien (114, 115) sont droites ou courbées, de préférence sont orientées en forme de S, et que les rainures (10) sont orientées perpendiculairement (114) et/ou obliquement (115) par rapport au sens d'extension longitudinale.

9. Tête de masque laryngé (1) selon la revendication 7,
**caractérisée en ce que** les parois de soutien (115) inclinées différemment par rapport au sens d'extension longitudinale de la rainure (10) traversent celle-ci.

10. Tête de masque laryngé (1) selon la revendication 2,
**caractérisée en ce que** les parois latérales (101) de la rainure (10,20) et les organes de soutien élastique (11) peuvent être sélectionnés dans l'épaisseur de paroi correspondant à la force de ressort à obtenir.

11. Tête de masque laryngé (1) selon la revendication 1,
**caractérisée en ce que**, des deux côtés de l'axe médian (14) de la tête de masque laryngé (1), sont formées chaque fois deux rainures (10,20) ou plus orientées parallèlement les unes aux autres.

12. Tête de masque laryngé (1) selon la revendication 1,
**caractérisée en ce que**, dans le manchon (4) sont pratiquées au moins deux rainures (10) alignées l'une au-dessus de l'autre dans le sens ventral-dorsal.

13. Tête de masque laryngé (1) selon la revendication 1,
**caractérisée en ce que** les rainures (20) sont orientées avec un sens de pénétration sensiblement médial-latéral en forme de fer à cheval dans le sens d'extension du manchon (4), la pointe (1') du manchon (4) étant dépourvue de rainure.

14. Tête de masque laryngé (1) selon la revendication 1,
**caractérisée en ce que**, dans la zone dans laquelle le tube (2) traverse le manchon (4), se trouve au moins une rainure (10) pour l'adaptation de taille ventrale-dorsale tandis que, ensuite, en direction de la pointe (1'), des deux côtés de la plaque de recouvrement, au moins une rainure (10) s'étend chaque fois dans le manchon pour l'adaptation de taille ventrale-dorsale.
